# EUROPÄISCHE PATENTSCHRIFT

(11) **EP 0 697 399 B1**
(45) Veröffentlichungstag und Bekanntmachung des Hinweises auf die Patenterteilung: **06.05.1998**
(21) Anmeldenummer: 95112480.9
(22) Anmeldetag: 09.08.1995
(51) Int. Cl.: C07C 231/08, C07C 231/12, C07C 233/03, C07C 233/05, C07C 233/58, C07C 233/65

(54) **Verfahren zur Herstellung von N-Alkenylcarbonsäureamiden**
Process for the preparation of N-alkenyl carboxylic acid amide
Procédé de préparation des N-alkenyl carboxamide

(30) Priorität: 19.08.1994 DE 4428901
(43) Veröffentlichungstag der Anmeldung: 21.02.1996
(73) Patentinhaber: BASF AKTIENGESELLSCHAFT, 67056 Ludwigshafen (DE)
(72) Erfinder: Rühl, Thomas, Dr., D-67227 Frankenthal (DE); Henkelmann, Jochem, Dr., D-68165 Mannheim (DE); Heider, Marc, Dr., D-67433 Neustadt (DE); Fiechter, Bernd, D-67459 Böhl-Iggelheim (DE)

(56) Entgegenhaltungen:
- EP-A- 0 184 074
- US-A- 5 023 375

## Beschreibung

Die vorliegende Erfindung betrifft ein neues Verfahren zur Herstellung von N-Alkenylcarbonsäureamiden der allgemeinen Formel I 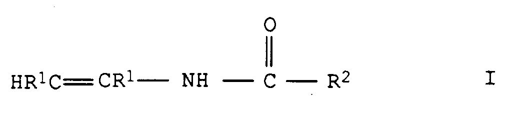 in der mindestens einer der Reste R¹ Wasserstoff bedeutet, der zweite Rest R¹ für Wasserstoff oder eine C₁-C₄-Alkylgruppe steht und der Rest R² für Wasserstoff, einen aliphatischen, cycloaliphatischen oder aromatischen Rest steht.

Die Verfahrensprodukte der Formel I sind gesuchte Zwischenprodukte. N-Alkenylcarbonsäureamide können in bekannter Weise polymerisiert werden und anschließend durch Hydrolyse in die entsprechenden Polyvinylamine überführt werden. Diese Polymere, insbesondere Polyvinylformamin, dienen beispielsweise zur Herstellung von Farbstoffen, pharmazeutischen Prozenten, Flockungsmitteln und Viskositäts-Stellmitteln in der Papierindustrie (Linhard et al., Das Papier 46/10A (1992), S. V 38-45).

N-Vinylformamid kann durch pyrolytische Spaltung bei ca. 300 bis 400°C aus Ethylidenformamid hergestellt werden. Ethylidenformamid kann beispielsweise unter Quecksilberkatalyse in saurer Lösung aus Formamid und Vinylacetat (DE-A 40 36 097) oder aus Formamid und Acetaldehyd in Gegenwart saurer Katalysatoren hergestellt werden (US-A 4 490 557).

Andere Herstellungswege verlaufen über Zwischenprodukte wie N-Acetylethylformamid (G. Parris, App. Cataly., 78 (1991) 65), N-Alkoxyethylformamid (DE-A 3 622 013, DE-A 3 520 829, US-A 4 670 531), N-Hydroxyethylformamid (US-A 4 567 300) und N-Cyanoethylformamid (DE-A 3 443 463). Diese Verbindungen eliminieren bei relativ hohen Temperaturen von ca. 300 bis 400°C Essigsäure, Alkohole, Wasser bzw. Cyanwasserstoff.

Die genannten Herstellungsverfahren sind zweistufig und erfordern einen thermischen Eliminierungsschritt. Sie sind somit technisch relativ aufwendig und führen aufgrund von Verlusten an Wertprodukt bei den erforderlichen hohen Reaktionstemperaturen nur zu unbefriedigenden Gesamtausbeuten. Es bestand daher die Aufgabe, ein Verfahren bereitzustellen, das die genannten Nachteile bekannter Verfahren vermeidet.

Demgemäß wurde ein Verfahren zur Herstellung von N-Alkenylcarbonsäureamiden der Formel I gefunden, das dadurch gekennzeichnet ist, daß man einen Carbonsäurealkenylester der allgemeinen Formel II 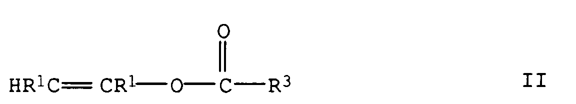 in der R¹ die oben angegebene Bedeutung hat und R³ für Wasserstoff, einen aliphatischen, cycloaliphatischen oder aromatischen Rest steht, und ein Carbonsäureamid der allgemeinen Formel III 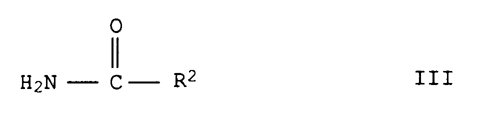 in der der Rest R² die oben angegebene Bedeutung hat, in Gegenwart einer Base umsetzt.

Die nachstehende Reaktionsgleichung verdeutlicht das beanspruchte Verfahren am Beispiel der Herstellung von N-Vinylformamid aus Vinylformiat und Formamid in Gegenwart von Triethylamin (Et = Ethyl):

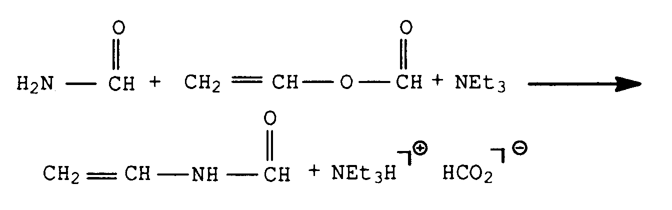

Im erfindungsgemäßen Verfahren wird formal eine Vinylgruppe aus einem Carbonsäurevinylester der Formel II auf ein Carbonsäureamid der Formel III übertragen.

Die Alkenylgruppe der Ester der Formel III kann einen C₁-C₄-Alkylrest wie Methyl, Ethyl, n-Propyl, iso-Propyl und n-Butyl tragen, bevorzugt sind jedoch Verbindungen, in denen die Reste R¹ für Wasserstoff stehen. Der Rest R³ in den Estern der Formel II steht für aliphatische Reste wie Alkyl- und Alkenylgruppen, die vorzugsweise 1 bis 40 Kohlenstoffatome tragen und geradkettig oder verzweigt sein können. Bevorzugt sind C₁-C₂₀-Alkylgruppe wie Methyl, Ethyl, n-Propyl, n-Butyl, tert.-Butyl und Stearyl. Weiterhin kann R³ für cycloaliphatische Reste, vorzugsweise mit 4 bis 7 Kohlenstoffatomen, stehen, z.B. Cyclopentyl und Cyclohexyl. Auch aromatische Reste wie Phenyl und Naphthyl kommen in Betracht, wobei diese unter den Reaktionsbedingungen inerte Substituenten wie Halogen und Alkoxy tragen können. Besonders bevorzugt steht R³ jedoch für Wasserstoff. Als Ausgangsverbindungen der Formel II seien genannt: Vinylformiat, Vinylacetat und Vinylstearat. Die Verbindungen der Formel II sind im Handel erhältlich oder nach bekannten Methoden herstellbar, beispielsweise durch Addition von Carbonsäuren an Acetylen.

Für den Rest R² der Amide der Formel III gilt das oben zum Rest R³ gesagte analog. Als Ausgangsverbindungen seien genannt: Formamid, Acetamid, Acrylamid. Auch diese Verbindungen sind im Handel oder nach bekannten Methoden erhältlich, z.B. durch Umsetzung von Carbonsäuren und Ammoniak unter Wasserabspaltung. Bevorzugtes Verfahrensprodukt ist N-Vinylformamid.

Die Ausgangsverbindungen werden in Gegenwart einer Base, bevorzugt eine Brönsted-Base, umgesetzt. Dazu kommen sowohl anorganische wie organische Basen in Betracht. Dabei handelt es sich im einzelnen um Carbonate und Hydrogencarbonate der Alkali- und Erdalkalimetalle wie Natriumcarbonat, Kaliumcarbonat und Natriumhydrogencarbonat, quartäre Ammoniumcarbonate wie Tetramethylammoniumcarbonat, Amide wie Alkalimetallamide, beispielsweise Natriumamid und Kaliumamid, Hydroxide wie Alkalimetallhydroxide, beispielsweise Lithiumhydroxid, Natriumhydroxid und Kaliumhydroxid, Alkalimetallcarboxylate wie Natriumacetat, Alkoholate wie Alkalimetallalkoholate, beispielsweise Natriummethanolat, Natriumethanolat, Kaliummethanolat und Kalium-tert.-butanolat. Kaliumhydroxid kann auch zusammen mit Kronenethern wie 18-Krone-6 verwendet werden.

Als Basen kommen weiterhin Amine wie Ammoniak sowie primäre, sekundäre und tertiäre Amine in Betracht, von denen die tertiären bevorzugt sind. Die Amine können aliphatische, cycloaliphatische oder aromatische Reste tragen, beispielsweise Trialkylamine wie Trioctylamin, Ethyldiisopropylamin, Diethylisopropylamin, Dimethylcyclohexylamin, Triethylamin, außerdem cyclische Amine wie 2,2,6,6-Tetramethylpiperidin, 1,4-Diazabicyclo[2.2.2]octan, 1,8-Diazabicyclo[5.4.0]undec-7-en, 1,5-Diazabicyclo[4.3.0]non-5-en, aliphatische und aromatische Reste tragende Amine wie 1,8-Bis(dimethylamino)-naphthalin und 4-Dimethylaminopyridin und heterocyclische Amine wie N-Alkylimidazole und N-Arylimidazole, z.B. N-Methylimidazol und N-Butylimidazol. Weiterhin kommen Amide wie Dialkylcarbonsäureamide, z.B. Dibutylformamid, in Betracht.

Das erfindungsgemäße Verfahren läßt sich auch in Gegenwart von basischen Ionenaustauschern, die in der Regel aus sulfonierten Styrol-Divinylbenzol-Copolymerisaten bestehen wie Amberlite®, Lewatit® und Puralit®, und in Gegenwart von basischen Zeolithen wie Hydrotalcit durchführen.

Pro Äquivalent Amid der Formel III können 0,1 bis 10, vorzugsweise 0,5 bis 2 Äquivalente des Esters der Formel II eingesetzt werden.

Die Basenmenge kann 0,1 bis 3 Äquivalente, bevorzugt 0,2 bis 1 Äquivalent pro Äquivalent Amid der Formel III betragen.

Obwohl die Umsetzung bevorzugt ohne Lösungsmittel ausgeführt wird, kann jedoch ein Lösungsmittel zugesetzt werden, z.B. aprotische Lösungsmittel wie Ether, z.B. Tetrahydrofuran, aromatische Kohlenwasserstoffe wie Toluol und Xylol, Ketone wie Aceton, weiterhin Acetonitril, Hexamethylphosphorsäuretriamid, Sulfolan und Dimethylsulfoxid. Die Menge liegt im allgemeinen bei 10 bis 30 Gew.-%, bezogen auf den Gesamtansatz.

Die Reaktionstemperatur liegt in der Regel bei 10 bis 100°C, bevorzugt bei 20 bis 60°C. Bevorzugt wird bei Normaldruck gearbeitet, es ist aber auch möglich, bei 0,01 bis 10 bar Reaktionsdruck zu arbeiten.

Die Reaktion kann kontinuierlich und diskontinuierlich ausgeführt werden. So können die Ausgangsverbindungen und die Base in einen Rührkessel gegeben und darin umgesetzt werden. Es ist auch möglich, die Ausgangsverbindungen und die Base in einem Rohrreaktor in Riesel- oder Sumpffahrweise umzusetzen.

In der Regel ist die Reaktion nach 5 Minuten bis 8 Stunden beendet.

Das so erhaltene Reaktionsgemisch kann in an sich bekannter Weise aufgearbeitet werden. Im allgemeinen wird das Produkt destillativ abgetrennt. Der Destillationssumpf kann zur Freisetzung der organischen Basen aus den bei der Reaktion anfallenden Salzen mit Laugen wie Natronlauge versetzt werden. Die freigesetzten Basen können anschließend extraktiv oder destillativ isoliert werden. Werden in der erfindungsgemäßen Umsetzung leichtflüchtige salzartige Verbindungen wie Formiate tertiärer Ammoniumverbindungen gebildet, können diese auch destillativ aufgearbeitet und in die entsprechenden Amine überführt werden. Die jeweils abgetrennten Basen können in die Reaktion zurückgeführt werden.

Das erfindungsgemäße Verfahren erlaubt die einstufige Herstellung von N-Alkenylamiden aus leicht zugänglichen Vorprodukten. Die Reaktion läßt sich technisch einfach gestalten und läuft unter milden Reaktionstemperaturen ab. Sie führt weiterhin zu einer hohen Ausbeute an den Verfahrensprodukten.

### Beispiele

### Allgemeine Reaktionsdurchführung

0,3 mol eines Esters der Formel II, 0,3 mol eines Amids der Formel III und 0,15 mol Base wurden bei 60°C und Normaldruck über 5 h umgesetzt.

Umsatz und Selektivität wurden gaschromatographisch bestimmt. In der folgenden Tabelle sind Einzelheiten zu den Umsetzungen angegeben:

| Bsp. | Amid | Ester | Base | Umsatz [%] | Selektivität [%] |
|---|---|---|---|---|---|
| 1 | Formamid | Vinylacetat | DMAP | 65 | 92 |
| 2 | Formamid | Vinylpropionat | DMAP | 62 | 95 |
| 3 | Acetamid | Vinylformiat | DMAP | 57 | 87 |
| 4 | Formamid | Vinylformiat | Triethylamin | 95 | 92 |
| 5 | Formamid | Vinylformiat | Dimethylcyclohexylamin | 92 | 87 |
| 6 | Formamid | Vinylformiat | Trioctylamin | 20 | 70 |
| 7 | Formamid | Vinylformiat | DABCO | 85 | 89 |
| 8 | Formamid | Vinylformiat | DBU | 73 | 86 |
| 9 | Formamid | Vinylformiat | DMAP | 97 | 94 |
| 10 | Formamid | Vinylformiat | Methylimidazol | 89 | 92 |
| 11 | Formamid | Vinylformiat | Dibutylformamid | 5 | 92 |
| 12 | Formamid | Vinylformiat | Ionentauscher Amberlite® IRA 420 | 91 | 87 |
| 13 | Formamid | Vinylformiat | Hydrotalcit | 80 | 78 |
| 14 | Formamid | Vinylformiat | Na₂CO₃ | 97 | 82 |
| 15 | Formamid | Vinylformiat | NaHCO₃ | 92 | 89 |
| 16 | Formamid | Vinylformiat | NaNH₂ | 97 | 65 |
| 17 | Formamid | Vinylformiat | NaOCH₃ | 98 | 73 |
| 18 | Formamid | Vinylformiat | KOH | 82 | 58 |
| 19 | Formamid | Vinylformiat | KOH/18-K-6 | 83 | 63 |
| DMAP = 4-Dimethylaminopyridin DABCO = 1,4-Diazabicyclo[2.2.2]octan DBU = 1,8-Diazobicyclo[5.4.0]undec-7-en 18-K-6= 18-Krone-6 | | | | | |

## Patentansprüche

1. Verfahren zur Herstellung von N-Alkenylcarbonsäureamiden der allgemeinen Formel I 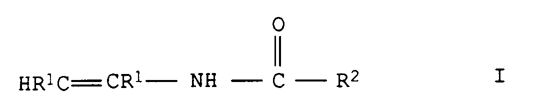 in der mindestens einer der Reste R¹ Wasserstoff bedeutet, der zweite Rest R¹ für Wasserstoff oder eine C₁-C₄-Alkylgruppe steht und der Rest R² für Wasserstoff, einen aliphatischen, cycloaliphatischen oder aromatischen Rest steht, dadurch gekennzeichnet, daß man einen Carbonsäurealkenylester der allgemeinen Formel II 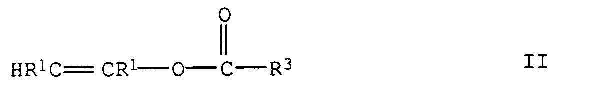 in der R¹ die oben angegebene Bedeutung hat und R³ für Wasserstoff, einen aliphatischen, cycloaliphatischen oder aromatischen Rest steht, und ein Carbonsäureamid der allgemeinen Formel III 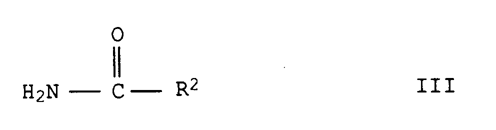 in der der Rest R² die oben angegebene Bedeutung hat, in Gegenwart einer Base umsetzt.

2. Verfahren nach Anspruch 1, dadurch gekennzeichnet, daß die Reste R¹ für Wasserstoff stehen.

3. Verfahren nach Anspruch 1 oder 2, dadurch gekennzeichnet, daß der Rest R² für Wasserstoff oder eine C₁-C₂₀-Alkylgruppe steht.

4. Verfahren nach den Ansprüchen 1 bis 3, dadurch gekennzeichnet, daß man N-Vinylformamid herstellt.

5. Verfahren nach den Ansprüchen 1 bis 4, dadurch gekennzeichnet, daß man die Umsetzung ohne Lösungsmittel vornimmt.

6. Verfahren nach den Ansprüchen 1 bis 5, dadurch gekennzeichnet, daß man als Base tertiäre Amine verwendet.

7. Verfahren nach den Ansprüchen 1 bis 6, dadurch gekennzeichnet, daß man 0,2 bis 1 Äquivalent Base pro Äquivalent Amid der Formel III verwendet.

8. Verfahren nach den Ansprüchen 1 bis 7, dadurch gekennzeichnet, daß man die Umsetzung bei 20 bis 60°C vornimmt.

## Claims

1. A process for the preparation of an N-alkenylcarboxamide of the formula I 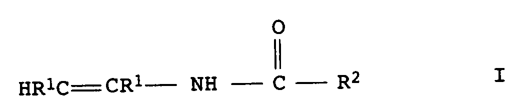 where at least one of the radicals R¹ is hydrogen, the second radical R¹ is hydrogen or C₁-C₄-alkyl and R² is hydrogen or an aliphatic, cycloaliphatic or aromatic radical, which comprises reacting an alkenyl carboxylate of the formula II 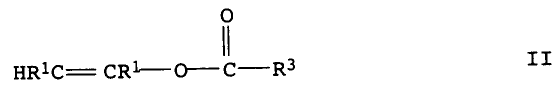 where R¹ has the abovementioned meanings and R³ is hydrogen or an aliphatic, cycloaliphatic or aromatic radical, and a carboxamide of the formula III 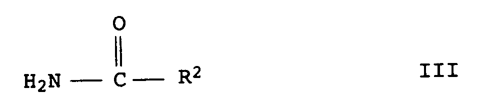 where R² has the abovementioned meanings, in the presence of a base.

2. A process as claimed in claim 1, wherein the radicals R¹ are hydrogen.

3. A process as claimed in claim 1 or 2, wherein R² is hydrogen or C₁-C₂₀-alkyl.

4. A process as claimed in any of claims 1 to 3, wherein N-vinylformamide is prepared.

5. A process as claimed in any of claims 1 to 4, wherein the reaction is carried out without solvent.

6. A process as claimed in any of claims 1 to 5, wherein the base used is a tertiary amine.

7. A process as claimed in any of claims 1 to 6, wherein from 0.2 to 1 equivalent of base is used per equivalent of amide of the formula III.

8. A process as claimed in any of claims 1 to 7, wherein the reaction is carried out at from 20 to 60°C.

## Revendications

1. Procédé de préparation de N-alcénylcarboxamides de formule générale I 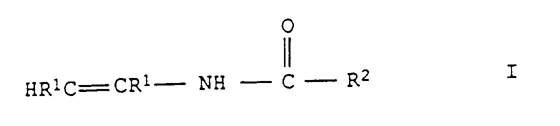 dans laquelle au moins un des restes R¹ représente un atome d'hydrogène, le deuxième reste R¹ représente un atome d'hydrogène ou un groupement alkyle en C₁-C₄ et le reste R² représente un atome d'hydrogène, un reste aliphatique, cycloaliphatique ou aromatique, caractérisé en ce que l'on fait réagir, en présence d'une base, un alcénylester d'acide carboxylique de formule générale II 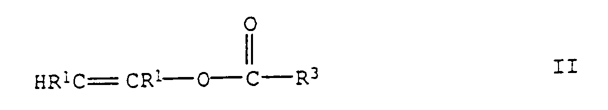 dans laquelle R¹ prend la signification susmentionnée et R³ est mis pour un atome d'hydrogène, un reste aliphatique, cycloaliphatique ou aromatique, avec un carboxamide de formule générale III 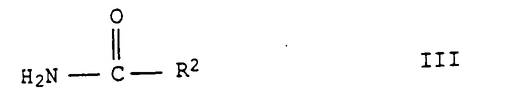 dans laquelle le reste R² prend la signification susmentionnée.

2. Procédé selon la revendication 1, caractérisé en ce que les restes R¹ sont mis pour des atomes d'hydrogène.

3. Procédé selon la revendication 1 ou 2, caractérisé en ce que le reste R² représente un atome d'hydrogène ou un groupement alkyle en C₁-C₂₀.

4. Procédé selon l'une quelconque des revendications 1 à 3, caractérisé en ce que l'on prépare du N-vinylformamide.

5. Procédé selon l'une quelconque des revendications 1 à 4, caractérisé en ce que l'on effectue la réaction sans solvant.

6. Procédé selon l'une quelconque des revendications 1 à 5, caractérisé en ce que l'on utilise des amines tertiaires en tant que base.

7. Procédé selon l'une quelconque des revendications 1 à 6, caractérisé en ce que l'on utilise 0,2 à 1 équivalent de base par équivalent d'amide de formule III.

8. Procédé selon l'une quelconque des revendications 1 à 7, caractérisé en ce que l'on effectue la réaction à 20-60°C.
